# EUROPEAN PATENT APPLICATION

(11) **EP 1 797 918 A1**
(43) Date of publication of application: **20.06.2007**
(21) Application number: 05027562.7
(22) Date of filing: 16.12.2005
(51) Int. Cl.: A61M 5/145

(54) **Syringe pump and docking station mountable on an IV pole**

(71) Applicant: Barak, Swi, Caesarea 38900 (IL)
(72) Inventor: Barak, Swi, Caesarea 38900 (IL)
(74) Representative: Kohler Schmid Möbus

(57) **Abstract**

A miniature syringe pump and a docking station mounted on an iv pole.

## Description

### Field and Background of the Invention

This invention relates to a syringe pump useful for administration of liquids to a patient by a medical pump for example from any size and type of syringe to a patient through a flexible tube.

Systems for administration of liquids to a patient is widely known. However, a variety of different pumps are available for propelling a drug to a patient, which may differ, among others, in the manner and principle in which they operate.

The present invention is concerned with two aspects for administrating a liquid to a patient. Its first aspect, the invention is concerned with a miniature mechanism for a syringe driver, which enable the system to be portable. The second aspect, the invention is concerned with control sensors for the use with a high precision liquid administration pump.

### Summary of the invention

The invention provides, by a first of its aspects, a miniature syringe pump for propelling drugs through a lumen of a flexible tube segment, the system comprising the miniature pump and a multi-purpose docking station. Once the pump was connected to the docking station, it's converted to be a stationary pump.
The Docking station has a male connector corresponding to a female connector on the pump, through the connector the syringe pump will:
a. Transmit the operation data to the docking station, that will display on a large and readable display.
b. Will charge the pump rechargeable batteries and will supply DC to the pump, while connected to mains.
c. Transmission of data to a central computer memory to create a file that can be tracked down.
d. Will enable connectivity to a central computer for data setting and control parameters.

The syringe pump mechanics comprises a screw bar and 2 guiding bars. The guiding bars will reduce the friction of the actuator to minimum and will assure smooth operation of the mechanics, but in parallel will be used for data transmission wires to the pump controller (micro controller) of the data from a micro switch mounted on the actuator, if the syringe is located on the actuator slot as required.

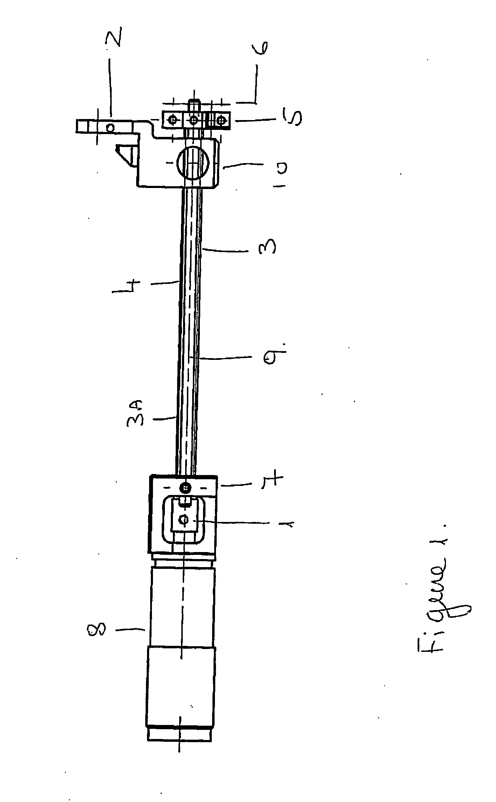

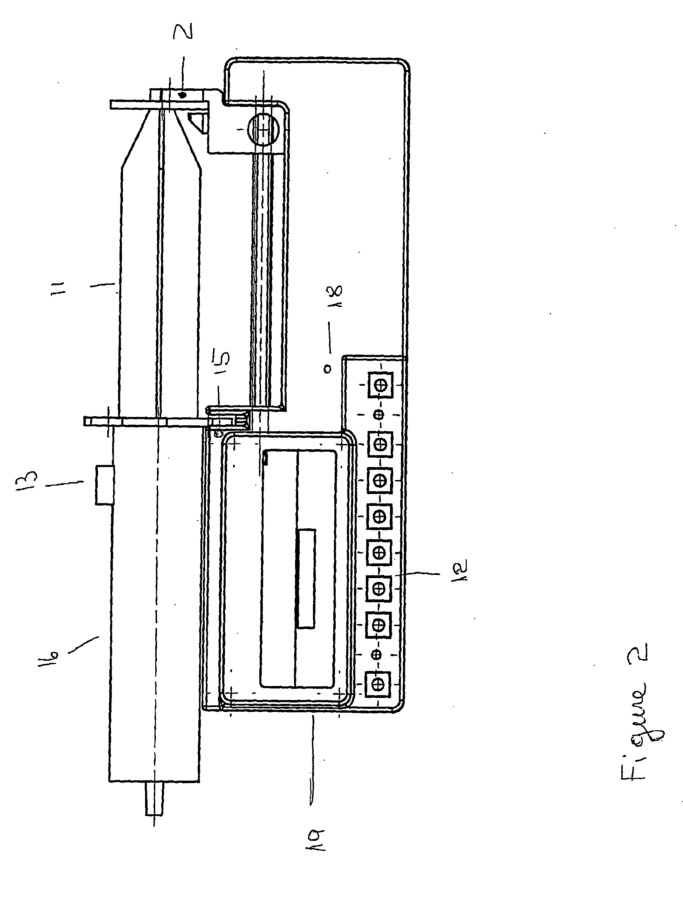

According to one preferred embodiment of the invention, the syringe pump has three sensors to detect the presence of the syringe and it's correct and safe located.
a. Sensor located on the actuator 2, where it's signals are transmitted through the guiding bars 3 and 3A on fig 1
b. Sensor located on the syringe location slot 15 on fig 2
c. Third sensor located on the syringe holder 13 connected to a linear potentiometer activated by a spring, not shown, to sense the diameter of the syringe.

By an improved design of the pump there is further provided with a door, not shown, with locking possibility, to protect any unauthorized access to the syringe.

By another preferred embodiment, the present invention includes a motor 8 fig 1 and an encoder 6 , for rotating the screw axis and control the location of the actuator 10.

According to a second aspect, of the present invention, there is provided a motor connected to a bearing (front bearing) 7 which is connected to a screw axis 9 and two guiding bars 3 and 3A that are connected to a second bearing (Rear Bearing) 5. The front bearing 7 has a magnet 1 on the motor axis that counts the number of motor revolutions which are compared with the results of two independent channels counted on the encoder mounted 6.

By another preferred embodiment of the present invention there is provided a motor 8 and a micro-controller, not shown, to control the motor revolution in order to get an improved linear delivery of the liquid and preventing pulsation effect. The micro-controller controls the motor revolutions by using the following algorithm:
a. the motor revolution is divided into a number of steps.
b. The micro-controller rotates the motor, sequentially from first step to the last step of each revolution, wherein each step or a group of steps are indicating the location of syringe actuator.
c. The pump output is controlled by three independent sensors which are comparing among them, results continuously.
d. The pump can detect the syringe size used using a linear potentiometer connected to the syringe holder 13. The syringe size can be sequentially detected during the pump work or can be used for calibration to obtain the syringe size and brand.

According to another aspect of the present invention, there is provided a dedicated flexible tube, with a pressure valve integrated, which will prevent free flow, of the syringe content if pressure is not generated by the syringe actuator.

### Detailed Description of Specific Embodiment

Reference is first being made to Fig 1 in which the mechanics of the syringe driver are shown. The motor 8 turns the screw axis 9 that initiate in both directions the movement of actuator 10.

The motor steps which initiate the actuator movement 10, are controlled by a magnet 1 and a hall effect sensor. Two independent sensors 6 are checking as well the motor movements.

The guiding bars 3 and 3A are stabilizing actuator 10, during movements, under pressure, but will as well conduct signals of sensor 2, indicating syringe location.

Figure 2 shows the pump 19, which can be connected to a docking station, not shown. The docking station having among other feature a LED display, is showing the most significant data on the LED display. Figure 2 shows three sensors that controls the syringe position number 15 controlling the syringe location in the syringe slot, number 13 senses the syringe diameter and number 2 the syringe plunger position. The operation led 18 will indicate the program status using a dual color led and number 12, a key board enable user to set/change the program.

## Claims

1. A miniature syringe pump and a docking station mounted on an iv pole.

2. The docking station that receive data from the pump through a corresponding connector and displays it on a large visible, from distance, display.

3. The docking station that receive data from the pump which has an LCD display, through a corresponding connector and converts the data and displays it on a LED display.

4. A docking station that receive data from a portable pump, of any type and kind, including infusion pumps, having an LCD (LIQUID CRYSTAL DISPLAY) through a corresponding connector, converts the data and displays it on a LED display.

5. A docking station that enable data collection from the pump.

6. A miniature mechanism that enable data transmission from a movable part through 2 guiding bars.

7. Disposable administration set with a pressure and one-way valve integrated.

8. A syringe pump with 3 independent sensors that compares motor revolutions.
